# EUROPEAN PATENT APPLICATION

(11) **EP 1 122 314 A1**
(43) Date of publication of application: **08.08.2001**
(21) Application number: 00200395.2
(22) Date of filing: 04.02.2000
(51) Int. Cl.: C12N 15/16, C07K 14/59, A01K 67/027

(54) **Method for the production of a protein**

(71) Applicant: Leja Research B.V., 2153 GN Nieuw Vennep (NL)
(72) Inventor: Vermeiden, Jan Pieter Willem, 3633 AG Vreeland (NL)
(74) Representative: Wittop Koning, Tom Hugo

(57) **Abstract**

Described is a method for the production of a heterodimeric protein by expressing one of the dimers in an animal, and the other dimer in another animal.

## Description

The invention relates to a method for the production of a protein, the protein comprising a first and a second peptide chain, the second chain being different from the first, the peptide chains being associated to each other.

In the art, proteins of the above-mentioned type are known. For biological activity, association of the two different peptide chains is a prerequisite. The first and second peptides may originate from the same gene, e.g. in the case of insulin, but are usually encoded by different genes, such as is the case for the protein hormones LH (lutenising hormone), FSH (follicle stimulating hormone), HCG (human chorion gonadopropine) and TSH (thyroid stimulating hormone). The latter all comprise an identical alpha chain and a hormone specific beta chain. It is to be understood that the protein according to the invention may comprise more than two different peptide chains or several identical peptide chains, as long as biological function of the protein requires association of two at least different peptide chains. In case the protein consists of two different peptide chains, such a protein is also known as a heterodimeric protein. Further examples of such proteins are e.g. all other heterodimeric biological active proteins like hergulin, integrins, activins and inhibins.

In this specification, a "peptide" is defined as a single, optionally glycosylated, amino acid chain, of which the molecular weight is not defined. Such peptide will be capable to associate with at least one other peptide to form the (heterodimeric) protein.

In the art, the proteins of the above-mentioned type are either produced from unmanupulated animals, as is the case for HCG that is isolated from urine of pregnant human individuals, and LH and FSH, being isolated from the urine of postmenopausal females. Another way to produce such proteins is by genetic modified cells, wherein both the first and second peptide chains are expressed, properly processed and associated to each other, forming the functional protein. At present, LH, FSH and HCG are prepared by genetically modified Chinese hamster ovarium cells (CHO).

However, the yield of genetically engineered proteins from cell lines is low, the isolation methods are difficult, resulting in a relative expensive protein product.

In the art, it is also known to produce genetically engineered proteins through transgenic animals. E.g. Nuijens, J.H. et al, J. Biol. Chem. 28:272 (13); 8802-7, 1997 describes the production of lactoferin from the milk of transgenic mice. The problem of the production of proteins by transgenic animals is the fact that the proteins, once expressed, may biologically be active in the animal and may result in undesired side-effects. Especially in the case of the production of protein hormones, e.g. those as described above, the transgenic animal may suffer from the expression of the said proteins. In genetic modified animals, producing a genetically engineered protein in the milk, about one percent of the expressed products appeared to "escape" from the milk into the body of the animal. In order to keep the transgenic animals in a healthy condition, high expression of the protein, i.e. of the peptides constituting with said protein, is therefore limited.

In order to obviate the above-mentioned draw-backs, the present invention relates to a method for the production of a protein, the protein comprising a first and a second peptide chain, the second chain being different from the first, the peptides chains being associated to each other, comprising the following steps
- bringing a first nucleic acid sequence, comprising
   - a first coding region, coding for the first peptide chain, and
   - a first control region, operably linked to the first coding region, controlling the expression of the first peptide chain in a first animal,
   into cells of the first animal and allowing expression of the first peptide chain in the animal by the said cells, the first peptide chain being exogenous for the first animal,
- isolating the first peptide chain from the animal, or its offspring,
- providing the second peptide chain in isolated form,
- reconstituting the protein by bringing together the first peptide chain isolated from the said animal with the second peptide under conditions allowing association of both peptide chains forming the protein.
By the said method, only one of the first or second peptide chains is produced by the animal, so that a functional protein cannot be formed, allowing high expression levels of the said peptide chain. The peptide chain is exogenous for the said animal; this means that the DNA coding for the said peptide chain is a DNA molecule that has been introduced into the animal by a process such as transformation, transfection, electropolation, gene bombardment or any other method known in the art. Please note that it is possible that the host cell into which the said DNA-molecule has been inserted may itself also naturally harbour sequences encoding the same or a similar peptide, which is encoded by the natural genome of the said animal. For example, when a specific animal, e.g. a cow, is used in this invention, it is recognized that the said animal naturally contains, in its genome, one or more sequences encoding e.g. the α- or β chain of LH, FSH, HCG or TSH. A molecule such as this is referred to as an "endogenous" DNA-molecule, encoding an endogenous peptide.

The exogenous nucleic acid sequence, also referred to as the transgenic sequence, that is to be introduced into cells of a specific animal, e.g. a cow, is preferably not of cow origin. However the transgenic sequence may encode for a peptide that is also enogenously produced by the animal.

The methods for bringing a nucleic acid sequence into an animal are known (see e.g. for a review Chan AWS, Cloning 1: 25-46, 1999), as well as the isolation of the transgenic products i.e. peptides, proteins (Niemann H et al, Transgenic Res. 8:237-47, 1999). In this way, at least one of the peptides of the (glyco) protein can be produced in a convenient manner with high yields. It is also possible, in case of the production of a protein, comprising more than two different peptide chains, to have the more than one of the said peptide chains produced by a single transgenic animal, as long as at least one of the peptide chains that are necessary for a functional protein product is not encoded on the transgenic nucleotide sequence. Preferably said nucleic acid sequence is stably introduced in the cells. This means that the transgenic nucleotide sequence is properly maintained, replicated and segregated during the cell cycle. Stable transformation may occur by chromosomal integration or by an extrachromosomal element, such as a suitable plasmid vector. Preferably the transgenic the nucleotide sequence is integrated in the genome of the animal cells.

In case a single transgenic animal is to produce more than one transgenic peptide, the sequence encoding said peptides may be located on a single nucleic acid, or on different transgenic sequences.

Preferably the nucleic acid sequences are brought into the animals in such a way that these sequences are propagated with the offspring of the said animals. In this specification, "offspring" means all animals, naturally breeded or cloned, having the transgenic animal wherein the transgenic nucleic acid has originally been introduced as ancestor. The technique of producing transgenic animals that are capable of producing transgenic offspring is known in the art (Chan AWS, supra). This is highly advantageous, as in that use the transgenic sequences are propagated, and the production of the protein is not limited to a single individual transgenic animal. Therefore, the method of the invention also encompasses the production of the protein from peptides, isolated from the offspring of the animal, wherein the transgenic nucleic acid sequence has originally been introduced.

"Reconstitution" as used herein refers to combining the different peptide chains of a protein under conditions that allow proper association and folding of the peptides resulting in a functional protein.

The skilled person will be aware of the fact that a transgenic peptide, produced according to the method of the present invention in an animal may, despite the fact that the said peptide is exogenous for the said animal, associate with a second peptide, endogenously produced by the said animal. In case such association leads to a biological active hybrid protein, care has to be taken that such hybrid protein does not lead to the above-mentioned drawback within the transgenic animal.

The transgenic peptide, produced according to the method of the present invention may also be a mutein that is capable to associate with its natural or also mutated counterpart, leading a biological active hybrid mutein.

The transgenic peptide or peptides can be isolated from the animal and be combined with the second peptide or the missing peptide(s) respectively to reconstitute the protein, resulting in a functional product. The second peptide chain may e.g. be produced by tissue culture or be obtained from natural sources.

However, it is highly preferred to have the second peptide chain produced by a second transgenic animal in a way as is described above. In this way, both peptide chains can be produced in high amounts, be isolated from the respective animals or their offspring, isolated and be brought together to reconstitute the protein.

It must be understood that the "first animal" and "second animal", as used herein, are different individuals; the first and second animals must not be of a different species, although this may be possible if desired (e.g. in view of the required glycosilation patterns that may be required for one or more of the transgenic peptide chains).

In a preferred embodiment of the method according to the invention, the protein is purified after reconstitution in order to provide the protein product of high purity that can be used in medicaments.

It is also possible to at least partially purify the different peptides after isolation and reconstitute the protein by bringing together the said peptides.

In a preferred embodiment, at least the first peptide chain is expressed by milk producing cells of a mammal and excreted in the milk by the said mammal. When the transgenic peptide(s) is present in the milk, the said peptide can easily be isolated from the mammal by milking the mammal. When both peptides (and optionally additional peptides in case the protein consists more than two different peptides) are produced in milk of different mammals, reconstitution may take place by just mixing the milk under appropriate conditions and ratio.

For expression of the required peptide chain by milk producing cells of the transgenic mammal, it is advantageous for the respective control region to comprise a casein promoter. The casein promoter has
been proven to be very suitable for this purpose, although other regulatory sequences can also be used (Brink, MF et al, Theriogenolgy 53: 139-48, 2000; Whitelaw CB et al, Transgenic Res. 1: 3-13, 1991). For optimal expression, the control region preferably comprises controlling sequences, such as e.g. promoter, enhancer, ribosomal binding site etc., preferably derived from the same species as the respective transgenic animal. Thus it is preferred to incorporate a cow casein promoter in the control region, when the peptide product is to be produced by a cow in her milk.

In another preferred embodiment of the invention, the first coding region codes for the α- chain of LH, FSH, HCG or TSH, and the second coding region for the corresponding β- chain of LH, FSH, HCG or TSH, respectively, or vice versa. By producing the α-chain of the said peptide hormones in the one animal and the β-chain in the other, these peptide hormones can easily be produced in high quantities. Preferably the α-chain and β-chain of the said hormones are of human origin to be optimally suitable for use in medicaments.

The invention further relates to a nucleic acid vector, comprising:
- a coding region, coding for a first peptide chain of a protein, the protein comprising a first and a second peptide chain, the second chain being different from the first, the peptides being associated to each other, and
- a control region, operably linked to the coding region, controlling the expression of the first peptide in an animal,
- the vector being void of a sequence resulting in the expression of the second peptide chain in the said animal.

Such a vector can be used to produce the transgenic animal according to the invention. It has however to be avoided that the said vector results in expression of both first and second peptide chains, which may lead to the above-mentioned drawbacks of a transgenic animal producing high level of functional protein.

In a preferred embodiment, said nucleic acid vector comprises
the coding region for the α- or β-chain of LH, FSH, HCG or TSH, preferably from human origin. The control region of the vector preferably comprises a casein promoter. The vector can be a DNA or RNA vector as is known in the art.

The invention also relates to an animal preferably a mammal, comprising in multiple cells, the first nucleic acid sequence according to the invention, the coding region thereof coding for an exogenous peptide. Preferably the nucleic acid sequence is propagated to the offspring of the said animal. By the presence of the said nucleic acid sequence, the exogenous peptide may be expressed in the animal and isolated for e.g. protein reconstitution purposes. In this specification, "animal" is to be understood as a higher animal, preferably a vertebrate, more preferably a mammal. Most preferred are mammals of which milk is easily isolated, i.e. cows, goats, sheep, rabbits etc..

The skilled person, aware of the above teaching will be capable of producing any protein such as a protein hormone, comprising different peptide chains that are needed for biological activity of the protein. Further, the choice of suitable animals can be easily assessed by the skilled person. In case the peptide to be produced needs glycosilation, an animal can be chosen that produces the said transgenic peptide with the required glycosilation pattern, or the glycosilation can be carried out in vitro after isolating the said peptide from the animal.

## Claims

1. Method for the production of a protein, the protein comprising a first and a second peptide chain, the second chain being different from the first, the peptide chains being associated to each other, **characterized by** the following steps:
- bringing a first nucleic acid sequence, comprising
- a first coding region, coding for the first peptide chain, and
- a first control region, operably linked to the first coding region, controlling the expression of the first peptide chain in a first animal,
into cells of the first animal and allowing expression of the first peptide chain in the animal by the said cells, the first peptide chain being exogenous for the first animal,
- isolating the first peptide chain from the animal, or its offspring,
- providing the second peptide chain in isolated form,
- reconstituting the protein by bringing together the first peptide chain isolated from the said animal with the second chain peptide under conditions allowing association of the both peptide chains forming the protein.

2. Method according to claim 1, **characterized in that** it comprises the following steps:
- bringing a second nucleic acid sequence, comprising
- a second coding region, coding for the second peptide chain, and
- a second control region, operably linked to the second control region, controlling the expression of the second peptide chain in a second animal,
into cells of the second animal, and allowing expression of the second peptide chain in the animal by the said cells, the second peptide chain being exogenous for the second animal,
- isolating the second peptide chain from the second animal, or its offspring, and
- reconstituting the protein by bringing together the first peptide chain isolated from the first animal or its offspring, with the second peptide isolated from the second animal or its offspring under conditions allowing association of both peptide chains forming the protein.

3. Method according to claim 1 or 2, **characterized in that** the protein is purified after reconstitution.

4. Method according to any of the preceding claims, **characterized in that** the first and second peptides are at least partially purified after isolation and subsequently reconstituted.

5. Method according to any of the preceding claims, **characterized in that** at least the first peptide chain is expressed by milk producing cells of a mammal and excreted in the milk by the said mammal.

6. Method according to any of the preceding claims, **characterized in that** at least one of the first and second control region comprises a casein promoter.

7. Method according to claims 2-6, **characterized in that** the first coding region codes for the α- chain of LH, FSH, HCG or TSH, and the second coding region for the corresponding β- chain of LH, FSH, HCG or TSH, respectively, or vice versa, the α-chain and the β- chain preferably being human sequences.

8. Method according to any of the preceding claims, **characterized in that** the nucleic acid sequences are brought into the respective animals in such a way that the said sequences are propagated with the offspring of the said animals.

9. Nucleic acid vector, comprising:
- a coding region, coding for a first peptide chain of a protein, the protein comprising a first and a second peptide chain, the second chain being different from the first, the peptides being associated to each other, and
- a control region, operably linked to the coding region, controlling the expression of the first peptide in an animal,
- the vector being void of a sequence resulting in the expression of the second peptide chain in the said animal.

10. animal, comprising in multiple cells the first nucleic acid sequence as defined in any of the preceding claims, the said nucleic acid sequence being exogenous for the said animal.
